# EUROPEAN PATENT SPECIFICATION

(11) **EP 2 262 534 B1**
(45) Date of publication and mention of the grant of the patent: **16.07.2014**
(21) Application number: 09720942.3
(22) Date of filing: 26.02.2009
(51) Int. Cl.: A61K 45/06, A61K 9/20, A61P 1/04

(54) **PALATABLE SOLID COMPOSITION COMPRISING ANTACID AND SALIVA STIMULANT**
SCHMACKHAFTE FESTSTOFFZUSAMMENSETZUNG MIT ANTACID UND EINEM SPEICHELSTIMULANS
COMPOSITION SOLIDE AGRÉABLE AU GOÛT COMPRENANT UN ANTIACIDE ET UN STIMULANT SALIVAIRE

(30) Priority: 10.03.2008 EP 08290223
(43) Date of publication of application: 22.12.2010
(73) Proprietor: Bayer Consumer Care AG, 4052 Basel (CH)
(72) Inventor: KABARADJIAN, Catherine, F-74100 Vétraz-Monthoux (FR)
(74) Representative: Albers, Markus
(86) International application number: PCT/EP2009/001353
(87) International publication number: WO 2009/112156

(56) References cited:
- US-A- 3 720 762
- US-A- 4 639 368
- US-A1- 2004 052 873
- US-A1- 2006 116 334

## Description

The present invention relates to a solid composition comprising at least one antacid and at least one neutral saliva stimulant, its process for preparation and its use for treating and/or preventing gastric disorders.

Saliva stimulants are generally used in food, buccal hygiene or gustatory preparations or in compositions for the treatment of nasal or oral tissue. There are several classes of saliva stimulants such as e.g. unsaturated alkamides, alkaloids or saliva stimulating peptides. An example is trans-pellitorin which is (2E,4E)-N-(2-methylpropyl)deca-2,4-dienamide, and is as a saliva stimulating aromatic substance used in food, buccal hygiene or gustatory preparations (WO 2004/043906). It is also used in compositions for the treatment of dry mouth (WO 2007/046890).

Antacids, e.g. aluminium hydroxide, magnesium hydroxide, calcium carbonate, magnesium carbonate or hydrotalcite, are described for providing rapid relief from the symptoms of excess of stomach acid by neutralizing the acid. They can be used for the treatment of heartburn, gastritis and other gastric disorders.

Different types of antacid compositions are known, e.g. tablets, chewable tablets, granules, liquids, suspensions, capsules, etc.. Most of the known solid antacid compositions disintegrate directly or form a suspension by chewing in the mouth in order to achieve a fast relief from heartburn. Fast disintegration can be achieved by chewing the solid composition and/or by adding particular disintegrants. However, solid antacid compositions when disintegrating or chewing in the mouth often show a chalky sensation in the mouth or under the teeth.

Surprisingly it is found that the composition according to the present invention show a palatable sensation without a chalky feeling in the mouth or under the teeth. The inventive composition is dispersed very fast in the saliva and can easily be swallowed even without water. Therefore the composition according to the present invention acts faster compared to a corresponding composition without a saliva stimulant and is much more palatable.

Subject of the present invention is a solid composition comprising at least one antacid and at least one neutral saliva stimulant.

Antacids are well-known and include magnesium hydroxide, magnesium carbonate, magnesium oxide, calcium carbonate, aluminium hydroxide, aluminium carbonate, aluminium phosphate, aluminium hydroxyl carbonate, dihydroxy aluminium sodium carbonate, aluminium magnesium glycinate, dihydroxy aluminium aminoacetate, dihydroxy aluminium aminoacetic acid, bismuth aluminate, bismuth carbonate, bismuth subcarbonate, bismuth subgallate, bismuth subnitrate, calcium phosphate, hydrated magnesium aluminate, magnesium aluminium silicates, magnesium glycinate, magnesium trisilicate, sodium hydrogen carbonate, hydrotalcite and mixtures thereof. Preference is given to hydtrotalcite, calcium carbonate, magnesium hydroxide, magnesium oxide, magnesium carbonate, sodium hydrogene carbonate and mixtures thereof. In particular a mixture of calcium carbonate, magnesium hydroxide and magnesium carbonate is used as antacid.

So called basic or heavy magnesium carbonate can also be used as antacid which are terms used in the European Pharmacopoeia and which correspond to a mixture of magnesium carbonate and magnesium hydroxide.

Saliva stimulants are generally known and comprise certain unsaturated alkamides such as e.g. pellitorines, spilantholes, or phenylalkanones e.g. shogaoles, gingerols, zingerones; alkaloides e.g. pilocarpine, and saliva stimulating peptides, e.g. substance p, tachykinins, or physalaemins, or others e.g. bethanechol chloride, affinin, capsaicine, dihydrocapsaicine, nordihydrocapsaicine, homocapsaicine, homodihydrocapsaicine, piperine, chavicine, paradol, Saanshool-I, Saanshool-II or Sanshoamide or vanillyl butyl ether acetate, nonylic acid vanillyl amide, 4- (1-menthoxymethyl) -2-phenyl-1, 3-dioxolan, 4- (1-menthoxymethyl) -2- (3' , 4' -dihydroxyphenyl) -1, 3-dioxolan, 4- (1-menthoxymethyl) -2- (2' -hydroxy-3' -methoxyphenyl) -1, 3- dioxolan, 4- (1-menthoxymethyl) -2-(4' -methoxyphenyl) -1, 3- dioxolan, 4- (1-menthoxymethyl) -2- (3' , 4' -methylenedioxy- phenyl) - 1, 3-dioxolan, 4- (1-menthoxymethyl) -2- (3' -methoxy- 4' -hydroxyphenyl) -1, 3-dioxolan as described e.g. in US2003464149A or WO 2007004740 A1, vanillyl alcohol n-butyl ether, vanillyl alcohol n-propyl ether, vanillyl alcohol isopropyl ether, vanillyl alcohol isobutyl ether, vanillyl alcolol n-amino ether, vanillyl alcohol isoamyl ether, vanillyl alcohol n-hexyl ether, vanillyl alcohol methyl ether, vanillyl alcohol ethyl ether, cinnamon oil, cinnamic aldehyde and phosphate derivatives of same as disclosed in US 20030215532A1, vanillin-1, 2-propylene glycol acetal, vanillin-1, 2-butylene glycol acetal, vanillin-1, 2-pentylene glycol acetal, vanillin-1, 2-hexylene glycol acetal, vanillin-1, 2-heptylene glycol acetal, vanillin-1, 2-octylene glycol acetal, vanillin-1, 2-nonylene glycol acetal, vanillin-1, 2-decylene glycol acetal, vanillin-1, 2-undecylene glycol acetal, vanillin-1-methoxyglycerol acetal, vanillin-1-ethoxyglycerol acetal, vanillin-1-propoxyglycerol acetal, vanillin-1-butoxyglycerol acetal, vanillin-1-pentoxyglycerol acetal, vanillin-1-hexyloxyglycerol acetal, vanillin-1- (2-ethyl) hexyloxyglycerol acetal, vanillin-1-heptyloxyglycerol acetal, and vanillin-1-octyloxyglycerol acetal. as described in WO 2007/004740 A1, alk-2-en-4-ynamides such as e.g. (2E)-N-(2-methylpropyl)dec-2-en-4-ynamide, (2E)-N-(2-methylbutyl)dec-2-en-4-ynamide, (2E)-1-(piperidin-1-yl)dec-2-en-4-yn-1-one, (2E)-1-(pyrrolidin-1-yl)dec-2-en-4-yn-1-one and their (2Z) isomers and - in case of asymmetric carbon atoms - their enantiomers as well as mixtures of at least two of the compounds as disclosed in DE 102006006123 A1, preferably pellitorines or spilantholes, especially preferred trans-pelletorin or spilanthole.

Saliva stimulants may cause warm or cool sensation on the mucous membranes and adherent skin.

Saliva stimulants that neither cause a distinctive warm felling nor a distinct cool feeling but a tingling or stinging sensation are called neutral. Examples for neutral saliva stimulants are spilanthols and pelletorins, whereby spilanthols are the active ingredients of Jambu Oleoresin and para cress (spilanthes species), and pelletorins are the active ingredients of pelletory root (*Anacyclus species*) i.e. N-(2-methylpropyl)amides selected from the group consisting of decanoic acid, 2E-decanoic acid, 2E,4Z-deca-2,4-dienoic acid, 2Z,4E-deca-2,4-dienoic acid, 2E,4Z,7Z-deca-2,4,7-trienoic acid, 3Z,5E-deca-3,5-dienoic acid and 3Z,5E,7Z-deca-3,5,7-trienoic acid such as trans-pellitorin ((2E,4E)-N-(2-methylpropyl)deca-2,4-dienamide) and cis-pelletorin ((2E,4Z)-N-(2-methylpropyl)deca-2,4-dienamide). Further neutral saliva stimulants are alk-2-en-4-ynamides such as e.g. (2E)-N-(2-methylpropyl)dec-2-en-4-ynamide, (2E)-N-(2-methylbutyl)dec-2-en-4-ynamide, (2E)-1-(piperidin-1-yl)dec-2-en-4-yn-1-one, (2E)-1-(pyrrolidin-1-yl)dec-2-en-4-yn-1-one and their (2Z) isomers and - in case of asymmetric carbon atoms - their enantiomers as well as mixtures of at least two of the compounds as disclosed in DE 102006006123 A1, saanshool-I, saanshool-II and sanshoamide as active ingredient of Japanes pepper extract (Zanthoxylum peperitum) and chavicin and piperine as active ingredients of black pepper extract (piper nigrum).

In a preferred embodiment of the inventive composition at least one neutral saliva stimulant is comprised.

In a preferred embodiment of the invention this neutral saliva stimulant is trans-pelletorin. Trans-pellitorin can advantageously be used in combination with at least one further N-(2-methylpropyl)amide selected from the group consisting of decanoic acid, 2E-decanoic acid, 2E,4Z-deca-2,4-dienoic acid, 2Z,4E-deca-2,4-dienoic acid, 2E,4Z,7Z-deca-2,4,7-trienoic acid, 3Z,5E-deca-3,5-dienoic acid and 3Z,5E,7Z-deca-3,5,7-trienoic acid. Preference is given to a mixture of trans-pellitorin and cis-pelletorin (2E,4Z)-N-(2-methylpropyl)deca-2,4-dienamide, preferably in a ratio of 80:20 by weight

In another embodiment of the invention the at least one neutral salivating stimulant or a further salivating stimulant can be comprised in plant or herb extracts as generally known to the person skilled in the art or described in US20070190090A1 or WO2007/004740 A1, such as e.g. extracts of para cress (*Spilanthes species, especially Spilanthes acmella* Murr. and *Spilanthes oleracea* L.), jaborandi (*Pilocarpus species, especially Pilocarpus jaborandi*), bloodroot (*Sanguinaria species, especially Sanguinaria canadensis*), blue flag (*Iris species, especially Iris versicolor*) , cayenne pepper (*Capsicum species, especially Capsicum minimum or Capsicum fructens*), centaury (*Centaurium species, especially Centaurium erythraea*), gentian (*Gentiana species, especially Gentiana lutea*), ginger (*Zingiber species, especially Zingiber officinale*), prickly ash or Japanese pepper (*Zanthoxylum species, especially Zanthoxylum americanum or Zanthoxylum piperitum*), senega (*Polygala species, especially Polygala senega*), black pepper (*Piper species, especially nigrum*), licorice (*Glycyrrhiza glabra*), gold root (*Heliopsis longipes (Compositae*)) purple coneflower (*Echinacea species, especially purpurea*), yerba santa (*Eriodictyon glutinosum*), bay berry (*Myrica species),* ginseng (*Panax ginseng),* kava (*Piper methysticum),* or kudzu (*Pueraria lobata),* knotweed or swamp smartweed (*Polygonum* species.,especially *Polygonum hydropiper*), onion or garlic (*Allium species*), radish (*Raphanus species*), horseradish (*Armoracia rusticana*) or wasabi (*Wasabia japonica* or *Cochlearia wasabi*), mustard (*Brassica species*), especially black mustard (*Brassica nigra*), brown mustard (*Brassica juncea*) or white or yellow mustard (*Sinapis species*., especially *Sinapis arvensis* and *Sinapis alba*), pellitory-root (*Anacyclus species,* especially *Anacylcus pyrethrum* L), German pellitory root (*Anacyclus officinalis, Hayne*), guinea pepper (*Aframomum especially,* especially *Aframomum melegueta*), pink pepper (*Schinus terebinthifolius* Raddi) or galangal (sand ginger or Thai ginger) (*Kaempferia galanga* or *Alpinia galanga*).

Alternatively, dried or freeze dried and optionally powdered plant parts can be added to the composition in amounts easily adjustable to the person skilled in the art.

Some of the preceding saliva stimulants cause a hot or warm feeling in the mouth such as e.g. vanillin-1, 2-propylene glycol acetal, vanillin-1, 2-butylene glycol acetal, vanillin-1, 2-pentylene glycol acetal, vanillin-1, 2-hexylene glycol acetal, vanillin-1, 2-heptylene glycol acetal, vanillin-1, 2-octylene glycol acetal, vanillin-1, 2-nonylene glycol acetal, vanillin-1, 2-decylene glycol acetal, vanillin-1, 2-undecylene glycol acetal, vanillin-1-methoxyglycerol acetal, vanillin-1-ethoxyglycerol acetal, vanillin-1-propoxyglycerol acetal, vanillin-1-butoxyglycerol acetal, vanillin-1-pentoxyglycerol acetal, vanillin-1-hexyloxyglycerol acetal, vanillin-1- (2-ethyl) hexyloxyglycerol acetal, vanillin-1-heptyloxyglycerol acetal, and vanillin-1-octyloxyglycerol acetal. as described in WO 2007/004740 A1 vanillyl butyl ether acetate, nonylic acid vanillyl amide, vanillyl butyl ether acetate, nonylic acid vanillyl amide, vanillyl alcohol n-butyl ether, vanillyl alcohol n-propyl ether, vanillyl alcohol isopropyl ether, vanillyl alcohol isobutyl ether, vanillyl alcolol n-amino ether, vanillyl alcohol isoamyl ether, vanillyl alcohol n-hexyl ether, vanillyl alcohol methyl ether, vanillyl alcohol ethyl ether, cinnamon oil, cinnamic aldehyde and phosphate derivatives of same as disclosed in US 20030215532A1, 4- (1-menthoxymethyl) -2-phenyl-1, 3-dioxolan, 4- (1-menthoxymethyl) -2- (3' , 4' -dihydroxyphenyl) -1, 3-dioxolan, 4- (1-menthoxymethyl) -2- (2' -hydroxy-3' -methoxyphenyl) -1, 3- dioxolan, 4- (1-menthoxymethyl) -2-(4' -methoxyphenyl) -1, 3- dioxolan, 4- (1-menthoxymethyl) -2- (3' , 4' -methylenedioxy- phenyl) - 1, 3-dioxolan, 4- (1-menthoxymethyl) -2- (3' -methoxy- 4' -hydroxyphenyl) -1, 3-dioxolan as described e.g. in US 5,545,424, US2003464149A or WO 2007004740 A1, or plants or plant extracts comprising any such compounds. They can be advantageously mixed with

at least one cooling saliva stimulant such as n-N-substituted-p-menthane-3-carboxamides, acyclic tertiary and secondary carboxamides, 3-1-menthoxy propan-1,2-diol as described in WO 97/06695 or menthol, menthone, camphor, pulegol, isopulegol, cineole, mint oil, peppermint oil, spearmint oil, eucalyptus oil, 3-1-menthoxypropane-1, 2-diol, N-alkyl-p-menthane-3-carboxamide, 3-1-menthoxy-2-methylpropane-1, 2-diol, p-menthane-3, 8-diol, 2-1-menthoxyethan-1-ol, 3-1-menthoxypropan-1-ol, 4-1-menthoxybutan-1-ol, menthyl 3-hydroxybutanate, menthyl lactate, menthone glycerin ketal, 2- (2-1-menthyloxyethyl) ethanol, menthyl glyoxylate, 1- (2-hydroxy-4-methylcyclohexyl) ethanone, N-methyl-2 , 2-isopropylmethyl-3-methylbutanamide, menthyl 2-pyrrolidone-5-carboxylate, monomenthyl succinate, alkali metal salts of monomenthyl succinate, alkaline earth metal salts of monomenthyl succinate, monomenthyl glutarate, alkali metal salts of monomenthyl glutarate, alkali earth metal salts of monomenthyl glutarate, N- [ [5-methyl-2- (1-methylethyl) - cyclohexyl] carbonyl] glycine, p-menthane-3-carboxylic acid glycerol ester, Menthol propylene glycol carbonate; Menthol ethylene glycol carbonate, and 6-isopropyl-3, 9-dimethyl-1, 4- dioxaspiro [4.5] decan-2-one as described in WO 98/47482, WO 2007004740 A1, or US2003464149A, other cooling saliva stimulants being disclosed in U.S. Patent Nos. 7,030,273 and 6,780,443 with respect to the cooling saliva stimulants and their preparation or sources,
and/or
at least one other suitable conventional flavor e.g. fruit flavors such as apple, acerola, apricot, black currant, blackberry, bloodorange, blueberry, boysenberry, cherry, cranberry, elderberry, grapefruit, kiwi, lemon, lime, mango, maracuja, melon, mulberry, orange, papaya, peach, pear, pineapple, pitaya, plum, raspberry, red currant, strawberry- alone or in mixtures and/or
with at least one other flavors such as e.g. almond, amaretto, caramel, cardamom, chocolate, cinnamon, coco, ginger, hazelnut, hibiscus, honey, linden blossoms, lemongrass, maple, pumpkin, rhubarb, rose blooms, thyme, vanilla, walnut, or the like - alone or in mixtures. Such conventional flavours are known and commercially available.

In another preferred embodiment the at least one neutral saliva stimulant is combined with at least one warming saliva stimulant or with at least one cooling saliva stimulant, in another preferred embodiment the at least one neutral saliva stimulant is combined with at least one warming saliva stimulant and at least one cooling saliva stimulant. In a further preferred embodiment the at least one neutral saliva stimulant is combined with at least one warming saliva stimulant and at least one cooling saliva stimulant and furthermore with at least one conventional fruit flavor and/or other flavor as defined above.

Preferably the saliva stimulant is comprised in mixtures to result in combinations such as e.g. raspberry-chili, cinnamon-orange or -apple, strawberry-rhubarb-pink pepper, pumpkin-papaya-cayenne, apricot-maracuja-guinea pepper, ginger-peach, -plum or -lemon, maple-walnut vanilla, chocolata-chili optionally with orange, chocolate-mint, coco-pineapple-galangal (spicy tropical fruit,), etc.

According to the present invention the amount of the antacid in the composition is in a dosage range of from about 5 to 160 ANC, preferably from 5 to 20 ANC (ANC = acid neutralizing capacity, determined according to the Current British Pharmacopoeia (method for hydrotalcite), and for Carbonates or to Rossett Rice test (method for calcium and magnesium carbonates). The amount of the antacid preferably used in the composition is from 50 to 2000 mg.

Calcium carbonate as antacid is preferably used in the composition in an amount from 100 to 2000 mg, more preferably from 350 to 1500 mg, most preferably from 500 to 1000 mg.

Heavy or basic magnesium carbonate as antacid is preferably used in the composition in an amount from 50 to 2000 mg, more preferably from 60 to 1000 mg. Most preferably heavy or basic magnesium carbonate as antacid is used in amount from 60 to 100 mg when combined with a further antacid.

Sodium hydrogen carbonate as antacid is preferably used in the composition in an amount from 100 to 2000 mg, more preferably from 350 to 1500 mg, most preferably from 500 to 1000 mg.

Hydrotalcite as antacid is preferably used in the composition in an amount from 100 to 2000 mg, more preferably from 350 to 1500 mg, most preferably from 500 to 1100 mg.

The content of the saliva stimulant can be easily adapted by a person skilled in the art and may vary dependent of the nature and intensity of the saliva stimulant and/or its combinations.

The saliva stimulant can be used in the composition in an amount from 0.00001 % to 0.05 %, preferably from 0.0001 % to 0.001 %, most preferably from 0.0004 % to 0.006 % by weight of the total composition.

In a preferred embodiment trans-pelletorin is used as saliva stimulant. Trans-pellitorin can also be used in the composition in an amount from 0.0001 mg up to 1 mg, preferably from 0.001 mg up to 0.1 mg, more preferably from 0.005 mg up to 0.08 mg.

As a source for trans-pellitorin the marketed product Optaflow ® (Symrise AG) can be used which contain trans-pellitorin in an amount of from 0.1 % up to 0.4 %. Optaflow ® can be used in the composition in an amount of from 1 mg up to 50 mg, preferably from 3 mg up to 30 mg, more preferably from 5 mg up to 20 mg. Optaflow ® can also be used in the composition in an amount of from 0.1 % up to 4 %, preferably from 0.3 % up to 3 %, more preferably from 0.4 % up to 2 % by weight of the total composition.

The composition according to the invention is administered orally one or more, preferably up to three, more preferably up to two times per day. With each administration the number of dosage forms taken in at the same time should not exceed two.

The composition according to the present invention can be used for treating or preventing gastric disorders e.g. acid indigestion, heartburn, hyperacidity and gastritis, preferably acute gastritis, in a patient. A patient, for the purpose of this invention, is a mammal, including a human.

The invention further comprises the use of the composition in a method of treating or preventing gastric disorders or diseases such as acid indigestion, heartburn, hyperacidity or gastritis by orally administering the inventive composition at least once daily up to thrice, preferably twice daily.

Nevertheless, it may in some cases be advantageous to deviate from the amounts specified, depending on body weight, type and degree of the disorder, individual behaviour toward the active ingredient, type of preparation and time or interval over which the administration is effected. For instance, less than the aforementioned minimum amounts may be sufficient in some cases, while the upper limit specified has to be exceeded in other cases.

The composition according to the invention comprises suitable administration forms which deliver the compounds of the invention and which include solid formulations such as tablets, tablets which disintegrate rapidly in the oral cavity (orodispersible tablets), fizzy tablets, powders, sachets, granules, pellets, chewable tablets, dispersible tablets and chewing gum.

Preference is given to powders, granules, pellets, orodispersible tablets, chewable tablets, dispersible tablets, fizzy tablets and chewing gum.

The most preferred embodiment of the present invention is a powder form containing at least one antacid and at least one saliva stimulant, preferably trans-pellitorin.

Further ingredients of the oral dosage form are those which are accepted for pharmaceuticals and nutritional supplements and physiologically unobjectionable, for example: as fillers cellulose derivatives (e.g. microcrystalline cellulose), sugars (e.g. lactose), sugar alcohols (e.g. mannitol, sorbitol), inorganic fillers (e.g. calcium phosphates), binders (e.g. polyvinylpyrrolidone, gelatin, starch derivatives and cellulose derivatives), and all other excipients required to produce formulations of pharmaceuticals and nutritional supplements of the desired properties, e.g. lubricants (magnesium stearate), e.g. disintegrants (e.g. crosslinked polyvinylpyrrolidone, sodium carboxymethylcellulose), e.g. wetting agents (e.g. sodium lauryl sulphate), e.g. release-slowing agents (e.g. cellulose derivatives, polyacrylic acid derivatives), e.g. coloured pigments or flavors.

Excipients for pharmaceuticals and nutritional supplements familiar to the skilled person are also described for example in the following handbook: "Handbook of Pharmaceutical Excipients", Rowe R.C., Sheskey P.J. & Weller, P.J., American Pharmaceutical Association, Washington, 4th edition 2003.

In another embodiment of the present invention the formulation is sugar-free. Surprisingly it was found that no sugar was required in the inventive formulation in order to achieve a palatable formulation which is readily dispersed in saliva and can be swallowed without additional intake of water. It was especially surprising that no chalky feeling in the mouth or under the teeth was experienced.

The dosage forms mentioned herein are produced by general standard processes. E.g. powders or granules can be produced by mixing the active ingredients together with the excipients to form a blend which is optionally granulated. Also different blends containing different ingredients and excipients can be premixed and combined to a final blend which is then optionally granulated. Afterwards the blend or the granule can optionally be pressed into tablets.

### Examples:

### Example 1:

### A)

Powder composition for a unit dose of approx. 1270 mg.

| **Names of ingredients** | **Quantity (mg)** | **Function** |
|---|---|---|
| **ACTIVE INGREDIENTS** | | |
| Calcium carbonate | 680.0 | antacid |
| Heavy magnesium carbonate | 80.0 | antacid |

| **EXCIPIENTS** | | |
|---|---|---|
| Sorbitol | 400.0 | bulking agent / sweetener |
| Pregelatinised maize starch | 20.0 | binding agent |
| Potato starch | 13.0 | disintegrating agent |
| Talc | 35.5 | lubricant |
| Magnesium stearate | 10.7 | lubricant |
| Light liquid paraffin | 5.0 | lubricant |
| Spearmint flavour | 10.0 | flavour |
| Saccharin sodium | 0.8 | sweetener |
| Optaflow ® A SD | 15.0 | flavour |

### B)

Powder composition for a unit dose of approx. 1010 mg.

| **Names of ingredients** | **Quantity (mg)** | **Function** |
|---|---|---|
| **ACTIVE INGREDIENTS** | | |
| Calcium carbonate | 500.0 | antacid |

| **EXCIPIENTS** | | |
|---|---|---|
| Sorbitol | 400.0 | bulking agent / sweetener |
| Pregelatinised maize starch | 20.0 | binding agent |
| Potato starch | 13.0 | disintegrating agent |
| Talc | 35.5 | lubricant |
| Magnesium stearate | 10.7 | lubricant |
| Light liquid paraffin | 5.0 | lubricant |
| Spearmint flavour | 10.0 | flavour |
| Saccharin sodium | 0.8 | sweetener |
| Optaflow ® A SD | 15.0 | flavour |

Optaflow ® A SD is commercially available and comprises trans-pellitorin.

### Manufacturing process for the powder of example 1 A and B

All ingredients but without Optaflow ® A SD are mixed together and a fluid bed aqueous granulation is conducted. The granulate is then dried and sieved and is mixed fmally with Optaflow ® A SD.

### Example 2:

### A)

Powder composition for a unit dose ofapprox. 1255 mg.

| **Names of ingredients** | **Quantity (mg)** | **Function** |
|---|---|---|
| **ACTIVE INGREDIENTS** | | |
| Calcium carbonate | 680.0 | antacid |
| Heavy magnesium carbonate | 80.0 | antacid |

| **EXCIPIENTS** | | |
|---|---|---|
| Sorbitol | 400.0 | bulking agent / sweetener |
| Pregelatinised maize starch | 20.0 | binding agent |
| Potato starch | 13.0 | disintegrating agent |
| Talc | 35.5 | lubricant |
| Magnesium stearate | 10.7 | lubricant |
| Light liquid paraffin | 5.0 | lubricant |
| Spearmint flavour | 10.0 | flavour |
| Saccharin sodium | 0.8 | sweetener |
| Trans-pelletorin | 0,05 | flavour |

### B)

Powder composition for a unit dose of approx. 995 mg.

| **Names of ingredients** | **Quantity (mg)** | **Function** |
|---|---|---|
| **ACTIVE INGREDIENTS** | | |
| Calcium carbonate | 500.0 | antacid |

| **EXCIPIENTS** | | |
|---|---|---|
| Sorbitol | 400.0 | bulking agent / sweetener |
| Pregelatinised maize starch | 20.0 | binding agent |
| Potato starch | 13.0 | disintegrating agent |
| Talc | 35.5 | lubricant |
| Magnesium stearate | 10.7 | lubricant |
| Light liquid paraffin | 5.0 | lubricant |
| Spearmint flavour | 10.0 | flavour |
| Saccharin sodium | 0.8 | sweetener |
| Trans-pelletorin | 0,05 | flavour |

### Manufacturing_process for the powder of examples 2 A and B

All ingredients but trans-pelletorin which is added to the granulation solvent are mixed together and a fluid bed aqueous granulation is conducted. The granulate is then dried and sieved.

### Example 3:

Powder composition for a unit dose of approx. 1505 mg.

| **Names of ingredients** | **Quantity (mg)** | **Function** |
|---|---|---|
| **ACTIVE INGREDIENTS** | | |
| Calcium carbonate | 500.0 | antacid |

| **EXCIPIENTS** | | |
|---|---|---|
| Sorbitol | 400.0 | bulking agent / sweetener |
| Pregelatinised maize starch | 20.0 | binding agent |
| Potato starch | 13.0 | disintegrating agent |
| Talc | 35.5 | lubricant |
| Magnesium stearate | 10.7 | lubricant |
| Spearmint flavour | 10.0 | flavour |
| Saccharin sodium | 0.8 | sweetener |
| Trans-pelletorin | 0,05 | flavour |

### Manufacturing process for the powder of example 3

All ingredients but trans-pelletorin which is added to the granulation solvent are mixed together and a fluid bed aqueous granulation is conducted. The granulate is then dried and sieved..

### Example 4:

Powder composition for a unit dose of approx. 1490 mg.

| **Names of ingredients** | **Quantity (mg)** | **Function** |
|---|---|---|
| **ACTIVE INGREDIENTS** | | |
| Sodium hydrogen carbonate | 1000 | antacid |

| **EXCIPIENTS** | | |
|---|---|---|
| Sorbitol | 400.0 | bulking agent / sweetener |
| Pregelatinised maize starch | 20.0 | binding agent |
| Potato starch | 13.0 | disintegrating agent |
| Talc | 35.5 | lubricant |
| Magnesium stearate | 10.7 | lubricant |
| Spearmint flavour | 10.0 | flavour |
| Saccharin sodium | 0.8 | sweetener |
| Optaflow ® A SD | 15.0 | flavour |

Optaflow ® A SD is commercially available and comprises trans-pellitorin.

### Manufacturing process for the powder of example 4

All ingredients but without Optaflow ® A SD are mixed together and a fluid bed aqueous granulation is conducted. The granulate is then dried and sieved and is mixed finally with Optaflow ® A SD.

### Example 5:

Powder composition for a unit dose of approx. 1490 mg.

| **Names of ingredients** | **Quantity (mg)** | **Function** |
|---|---|---|
| **ACTIVE INGREDIENTS** | | |
| Sodium hydrogen carbonate | 1000 | antacid |

| **EXCIPIENTS** | | |
|---|---|---|
| Sorbitol | 400.0 | bulking agent / sweetener |
| Pregelatinised maize starch | 20.0 | binding agent |
| Potato starch | 13.0 | disintegrating agent |
| Talc | 35.5 | lubricant |
| Magnesium stearate | 10.7 | lubricant |
| Spearmint flavour | 10.0 | flavour |
| Saccharin sodium | 0.8 | sweetener |
| Trans-pelletorin | 0,05 | flavour |

### Manufacturing process for the powder of example 5

All ingredients but trans-pelletorin which is added to the granulation solvent are mixed together and a fluid bed aqueous granulation is conducted. The granulate is then dried and sieved..

### Example 6:

Powder composition for a unit dose of approx. 1180 mg.

| **Names of ingredients** | **Quantity (mg)** | **Function** |
|---|---|---|
| **ACTIVE INGREDIENTS** | | |
| Hydrotalcite Fine Granules | 500.0 | antacid |

| **EXCIPIENTS** | | |
|---|---|---|
| Sorbitol | 266.0 | bulking agent / sweetener |
| Mannitol | 360.0 | Bulking agent |
| Citric Acid | 20.0 | Flavour enhancer |
| Aspartame | 14.0 | sweetener |
| Magnesium stearate | 10.0 | lubricant |
| Evogran Orange | 5.0 | flavour |
| Optaflow ® A SD | 5.0 | flavour |

### Manufacturing process for the powder of example 6

All ingredients are mixed, sieved and filled in stick-packs.

Hydrotalcite Fine Granules are provided by Kyowa Chemical Industry Co., Ltd.

### Results:

The compositions of examples 1 to 6 show a palatable sensation without a chalky feeling in the mouth or under the teeth. They are dispersed very fast in the saliva and can easily be swallowed even without water. The compositions of examples 1 to 6 act faster compared to a corresponding composition without trans-pellitorin. It is surprising that the compositions of examples 1 to 6 are as palatable although they do not contain any sugar. In example 6 the chalky taste of hydrotalcite is reduced and the composition can be used without water.

## Claims

1. Palatable solid composition comprising at least one antacid and at least one neutral saliva stimulant for use in a method for treating or preventing gastric disorders or diseases such as acid indigestion, heartburn, hyperacidity or gastritis, where the at least one neutral saliva stimulant is a spilanthole, a pelletorin, a saanshool, a sanshoamide, chavicin or piperine.

2. Composition for use according to claim 1, wherein the at least one neutral saliva stimulant is pelletorin, preferably trans-pelletorin.

3. Composition for use according to any of claims 1 or 2, wherein the antacid is magnesium hydroxide, magnesium carbonate, magnesium oxide, calcium carbonate, aluminium hydroxide, aluminium carbonate, aluminium phosphate, aluminium hydroxyl carbonate, dihydroxy aluminium sodium carbonate, aluminium magnesium glycinate, dihydroxy aluminium aminoacetate, dihydroxy aluminium aminoacetic acid, bismuth aluminate, bismuth carbonate, bismuth subcarbonate, bismuth, subgallate, bismuth subnitrate, calcium phosphate, hydrated magnesium aluminate, magnesium aluminium silicates, magnesium glycinate, magnesium trisilicate, sodium bicarbonate, hydrotalcite or mixtures thereof

4. Composition for use according to any of claims 1 to 3, wherein the antacid is hydrotalcite, calcium carbonate, magnesium hydroxide, magnesium oxide, magnesium carbonate, sodium hydrogen carbonate or mixtures thereof.

5. Composition for use according to any of claims 1 to 4, wherein the antacid is a mixture of calcium carbonate, magnesium hydroxide and magnesium carbonate,

6. Composition for use according to any of claims 1 to 5, wherein the amount of the antacid is from 50 to 2000 mg.

7. Composition for use according to any of claims 1 to 6, wherein the amount of the saliva stimulant is from 0.00001 % to 0.05 % by weight of the total composition

8. Composition for use according to any of claims 1 to 7 which is a tablet, a tablet which disintegrate rapidly in the oral cavity (orodispersible tablets), a powder, a sachet, a granule, a pellet, a chewable tablet, a dispersible tablet, a fizzy tablet or a chewing gum.

9. Compositions for use according to any of claims 1 to 8, **characterized in that** they are sugar free.

## Patentansprüche

1. Wohlschmeckende feste Zusammensetzung, umfassend mindestens ein Antacidum und mindestens ein neutrales Speichelstimulans, zur Verwendung bei einem Verfahren zur Behandlung oder Prävention von Magenstörungen oder -erkrankungen wie erhöhter Salzsäureproduktion, Sodbrennen, Hyperazidität oder Gastritis, wobei es sich bei dem mindestens einen neutralen Speichelstimulans um ein Spilanthol, ein Pelletorin, ein Saanshool, ein Sanshoamid, Chavicin oder Piperin handelt.

2. Zusammensetzung zur Verwendung nach Anspruch 1, wobei es sich bei dem mindestens einen neutralen Speichelstimulans um Pelletorin, vorzugsweise trans-Pelletorin, handelt.

3. Zusammensetzung zur Verwendung nach Anspruch 1 oder 2, wobei sich bei dem Antacidum um Magnesiumhydroxid, Magnesiumcarbonat, Magnesiumoxid, Calciumcarbonat, Aluminiumhydroxid, Aluminiumcarbonat, Aluminiumphosphat, Aluminiumhydroxycarbonat, Dihydroxyaluminiumnatriumcarbonat, Aluminiummagnesiumglycinat, Dihydroxyaluminiumaminoacetat, Dihydroxyaluminiumaminoessigsäure, Bismutaluminat, Bismutcarbonat, Bismutsubcarbonat, Bismutsubgallat, Bismutsubnitrat, Calciumphosphat, Magnesiumaluminathydrat, Magnesiumaluminiumsilicat, Magnesiumglycinat, Magnesiumtrisilicat, Natriumhydrogencarbonat, Hydrotalcit oder Mischungen davon handelt.

4. Zusammensetzung zur Verwendung nach einem der Ansprüche 1 bis 3, wobei sich bei dem Antacidum um Hydrotalcit, Calciumcarbonat, Magnesiumhydroxid, Magnesiumoxid, Magnesiumcarbonat, Natriumhydrogencarbonat oder Mischungen davon handelt.

5. Zusammensetzung nach einem der Ansprüche 1 bis 4, wobei sich bei dem Antacidum um eine Mischung von Calciumcarbonat, Magnesiumhydroxid und Magnesiumcarbonat handelt.

6. Zusammensetzung zur Verwendung nach einem der Ansprüche 1 bis 5, wobei die Menge des Antacidums 50 bis 2000 mg beträgt.

7. Zusammensetzung zur Verwendung nach einem der Ansprüche 1 bis 6, wobei die Menge des Speichelstimulans 0,00001 bis 0,05 Gew.-%, bezogen auf die gesamte Zusammensetzung, beträgt.

8. Zusammensetzung zur Verwendung nach einem der Ansprüche 1 bis 7, bei der es sich um eine Tablette, eine in der Mundhöhle schnell zerfallende Tablette (orodispergierbare Tablette), ein Pulver, einen Beutel, ein Granulat, ein Pellet, eine Kautablette, eine dispergierbare Tablette, eine Brausetablette oder einen Kaugummi handelt.

9. Zusammensetzungen zur Verwendung nach einem der Ansprüche 1 bis 8, **dadurch gekennzeichnet, dass** sie zuckerfrei sind.

## Revendications

1. Composition solide palatable, comprenant au moins un antiacide et au moins un stimulant salivaire neutre, pour une utilisation dans une méthode de traitement ou de prévention de troubles ou de maladies gastriques tels que l'indigestion acide, le pyrosis, l'hyperacidité ou la gastrite, où le au moins un stimulant salivaire neutre est le spilanthol, la pelletorine, le sanshool, le sanshoamide, la chavicine ou la pipérine.

2. Composition pour une utilisation selon la revendication 1, dans laquelle le au moins un stimulant salivaire neutre est la pelletorine, préférablement la trans-pelletorine.

3. Composition pour une utilisation selon l'une quelconque des revendications 1 ou 2, dans laquelle l'antiacide est l'hydroxyde de magnésium, le carbonate de magnésium, l'oxyde de magnésium, le carbonate de calcium, l'hydroxyde d'aluminium, le carbonate d'aluminium, le phosphate d'aluminium, l'hydroxycarbonate d'aluminium, le dihydroxycarbonate d'aluminium et de sodium, le glycinate d'aluminium et de magnésium, l'aminoacétate de dihydroxyaluminium, l'acide dihydroxyaluminium aminoacétique, l'aluminate de bismuth, le carbonate de bismuth, le subcarbonate de bismuth, le subgallate de bismuth, le subnitrate de bismuth, le phosphate de calcium, l'aluminate de magnésium hydraté, les silicates de magnésium et d'aluminium, le glycinate de magnésium, le trisilicate de magnésium, le bicarbonate de sodium, l'hydrotalcite, ou des mélanges de ceux-ci.

4. Composition pour une utilisation selon l'une quelconque des revendications 1 à 3, dans laquelle l'antiacide est l'hydrotalcite, le carbonate de calcium, l'hydroxyde de magnésium, l'oxyde de magnésium, le carbonate de magnésium, l'hydrogénocarbonate de sodium, ou des mélanges de ceux-ci.

5. Composition pour une utilisation selon l'une quelconque des revendications 1 à 4, dans laquelle l'antiacide est un mélange de carbonate de calcium, d'hydroxyde de magnésium et de carbonate de magnésium.

6. Composition pour une utilisation selon l'une quelconque des revendications 1 à 5, dans laquelle la quantité d'antiacide va de 50 à 2000 mg.

7. Composition pour une utilisation selon l'une quelconque des revendications 1 à 6, dans laquelle la quantité de stimulant salivaire va de 0,00001% à 0,05% en poids de la composition totale.

8. Composition pour une utilisation selon l'une quelconque des revendications 1 à 7, qui est un comprimé, un comprimé qui se désintègre rapidement dans la cavité orale (comprimés orodispersibles), une poudre, un sachet, un granulé, une pastille, un comprimé croquable, un comprimé dispersable, un comprimé effervescent ou un chewing-gum.

9. Compositions pour une utilisation selon l'une quelconque des revendications 1 à 8, **caractérisées en ce qu'**elles sont dépourvues de sucre.
